# EUROPEAN PATENT APPLICATION

(11) **EP 4 039 297 A1**
(43) Date of publication of application: **10.08.2022**
(21) Application number: 21218130.9
(22) Date of filing: 29.12.2021
(51) Int. Cl.: A61M 11/00, A61M 15/00, A61M 15/02, A61M 15/08

(54) **DRUG DELIVERY DEVICE, METHOD OF CONTROLLING FLUID PLUME AND METHOD OF NASAL CAVITY INJECTION**

(30) Priority: 04.02.2021 US 202117167351
(71) Applicant: Funai Electric Co., Ltd., Daito, Osaka, 574-0013 (JP)
(72) Inventor: GIBSON, Bruce D., Lexington, KY 40508 (US); GIRI, Manish, Lexington, KY 40508 (US); JONES, Brian T., Lexington, KY 40508 (US); MARRA III, Michael A., Lexington, KY 40508 (US); MILGATE III, Robert W., Lexington, KY 40508 (US)
(74) Representative: Becker, Eberhard

(57) **Abstract**

A pharmaceutical drug delivery device (200) and method of using the pharmaceutical drug delivery device (200). The pharmaceutical drug deliver device (200) includes a cartridge body; a fluid outlet nozzle attached to the cartridge body; and a fluid jet ejection cartridge disposed in the cartrdige body, wherein the cartridge contains a liquid pharmaceutical drug and a fluid ejection head (202) containing a plurality of fluid ejection nozzles and associated fluid ejectors. A processor disposed on a logic board or fluid ejection head (202) is provided for executing a control algorithm to control the ejection head (202) to modify plume characteristics of fluid ejected from the ejection head (202) by controlling one or more of fluid jet firing frequency, burst length, and fluid jet firing burst delay.

## Description

### TECHNICAL FIELD

The disclosure is directed to inhalation drug delivery systems and in particular to modifying plume characteristics of fluid jet drug delivery systems for inhalation applications.

### BACKGROUND AND SUMMARY

Nasal spay devices have become important methods for delivering drugs to patients. Such nasal spray devices are more convenient to use than the administration of drugs through IV or injection. Nasal spray devices also provide higher bioavailability of drugs compared to oral administration of drugs. The absorption of drugs through nasal spray devices is more rapid compared to the absorption of drugs administered orally since drugs delivered by nasal spray devices directly enter the blood stream making their effect more immediate.

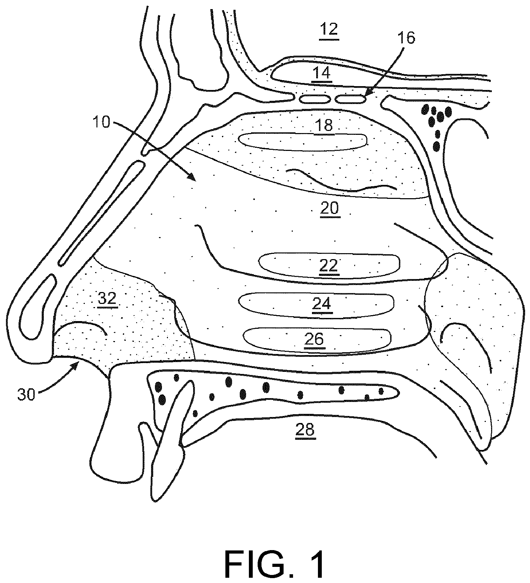

FIG. 1 is a cross sectional view, not to scale, of anatomy of a nasal cavity 10 of a person. A portion of the brain 12 is shown above the nasal cavity 10. An olfactory bulb 14 is disposed between the brain 12 and a cribriform plate 16. An olfactory mucosa 18 is below the cribriform plate 16. The nasal cavity also includes a superior turbinate 20, a middle turbinate 22, respiratory mucosa 24 and an inferior turbinate 26. Item 28 represents the palate. Injection of a pharmaceutical drug mist enters the nasal cavity 10 through the nostrils 30 and squamous mucosa 32. In order to achieve proper delivery of drugs to the blood stream using a nasal spray device, the drugs must be delivered to the respiratory mucosa 24 which is highly vascularized. Two areas that are highly vascularized are the olfactory region and the respiratory region. The respiratory region contains turbinates which increase the surface area available for drug absorption.

It is believed that smaller, lower velocity fluid droplets are best for deposition of drugs in the nasal cavity 10. Fluid droplets with high inertia will tend to move in a straight line and land at the point only where they are aimed. Fluid droplets with low inertia will be affected by air resistance and air currents and are more likely to float throughout the nasal cavity for more even drug delivery coverage.

Another apsect of nasal delivery of drugs that may increase deposition coverage is the plume angle of the fluid droplets. A wider plume angle is believed to provide greater mist formation and thus better coverage of drug delivery in the nasal cavity. Conventional methods for delivering drugs via the nasal cavity include medicine droppers, multi-spray bottles with spray tips, single-dose syringes with spray tips, and dry powder systems. Accordingly, conventional drug delivery devices are typically designed to deliver a specific drug to a nasal cavity and each device cannot be adapted for delivering a wide range of drugs via a nasal cavity route. Many of the conventional methods for nasal drug delivery rely on pressurized containers to inject a mist of fluid into the nasal cavity. Accordingly, the drug delivery devices are typically designed for a specific drug and cannot be adapted to administer a different drug.

Despite the availability of a variety of devices for delivering drugs via a nasal cavity route, there remains a need for a single nasal drug delivery device that can be tuned to deliver a variety of drugs over a range of velocities, fluid ejection times, and plume angles.

In view of the foregoing, an embodiment of the disclosure provide a pharmaceutical drug delivery device and method of using the pharmaceutical drug delivery device.

In one embodiment, the pharmaceutical drug deliver device includes a cartridge body; a fluid outlet nozzle attached to the cartridge body; and a fluid jet ejection cartridge disposed in the cartrdige body, wherein the cartridge contains a liquid pharmaceutical drug and a fluid ejection head containing a plurality of fluid ejection nozzles and associated fluid ejectors. A processor disposed on a logic board or the fluid ejection head is provided for executing a control algorithm to control the ejection head to modify plume characteristics of fluid ejected from the ejection head by controlling one or more operating parameters selected from (a) fluid jet firing frequency, (b) burst length, and (c) fluid jet firing burst delay.

In another embodiment, there is provided a method of controlling a fluid plume from a fluid ejection device for delivery of pharmaceutical drugs. The method includes providing the fluid ejection device having a cartridge body, a fluid outlet nozzle attached to the cartridge body and a fluid jet ejection cartridge disposed in the cartridge body. The fluid jet ejection cartridge contains a liquid pharmaceutical drug. A fluid ejection head containing a plurality of fluid ejection nozzles and associated fluid ejectors is attached to the fluid jet ejection cartridge and the fluid ejection head is in fluid flow communication with the fluid outlet nozzle. A processor is provided in electrical communication with the fluid ejection head. The processor is configered to execute a control algorithm to select one or more operating parameters selected from (a) fluid jet firing frequency, (b) burst length, and (c) fluid jet firing burst delay in order to modify fluid plume characteristics of fluid ejected from the ejection head through the fluid outlet nozzle. Upon activation of the fluid ejection device a pharmaceutical drug is delivered to a patient.

In another embodiment, there is provided a method for nasal cavity injection of pharmaceutical drugs. The method includes providing a fluid ejection device having a cartridge body, a fluid outlet nozzle attached to the cartridge body and a fluid jet ejection cartridge disposed in the cartridge body. The fluid jet ejection cartridge contains a liquid pharmaceutical drug. A fluid ejection head containing a plurality of fluid ejection nozzles and associated fluid ejectors is attached to the fluid jet ejection cartridge and the fluid ejection head is in fluid flow communication with the fluid outlet nozzle. A processor is provided in electrical communication with the fluid ejection head. The processor is configured to execute a control algorithm to select one or more operating parameters selected from (a) fluid jet firing frequency, (b) burst length, and (c) fluid jet firing burst delay in order to modify fluid plume characteristics of fluid ejected from the ejection head through the fluid outlet nozzle. The pharmaceutic drug is delivered to the nasal cavity of a person by activating the fluid ejection device.

In some embodiments, each fluid droplet ejected from the ejection head has volume ranging from about 2 to about 24 pL.

In some embodiments, each of the fluid ejectors has a firing frequency ranging from about 2 to about 20 KHz.

In some embodiments, the burst length ranges from about 20 to about 250 times per burst.

In some embodiments, the fluid jet firing burst delay ranges from about 0 milliseconds to about 15 milliseconds.

In some embodiments, the pharmaceutical drug is ejected from the device with a fluid plume angle ranging from about 25 to about 60 degrees.

In some embodiments, the pharmaceutical drug is ejected from the device with a fluid plume height ranging from about 10 to about 25 centimeters.

In some embodiments, the pharmaceutical drug is ejected from the device with a fluid jet length ranging from about 1 to about 25 centimeters from the fluid ejection head.

In some embodiments, the pharmaceutical drug is ejected with a plume characteristic that delivers the drug to turbinate areas of a nasal cavity of the patient.

In some embodiments, the pharmaceutical drug is ejected with a plume characteristic that evenly distributes the drug throughout a nasal cavity of the patient.

In some embodiments, the pharmaceutical drug is ejected with a plume characteristic that increases a drug dose delivery rate to the patient.

An advantage of the pharmaceutical drug delivery device described herein is that the device may be used for a wide variety of drugs having different fluid characteristics. The device is tunable by modifying certain fluid ejector characteristics in order to modify a plume angle, fluid jet length and/or plume height of fluid mist for nasal injection applications. Other features and advantage of the disclosed embodiments may be evident from the following drawings and detailed description.

### BRIEF DESCRIPTION OF THE DRAWINGS

FIG. 1 is a cross-sectional representation, not to scale, of a portion of a nasal cavity and skull of a person.
FIG. 2 is a cross-sectional view, not to scale of a pharmaceutical drug delivery device according to an embodiment of the disclosure.
FIGs. 3-5 are schematic illustrations of the pharmaceutical drug delivery device of FIG. 2 showing fluid jet and plume characteristics for different operating conditions.
FIG. 6 is a graphical representation of a change in fluid plume angle versus a change of ejector firing frequency for the pharmaceutical drug delivery device of FIG. 2.
FIG. 7 is a graphical representation of a change in fluid plume height versus a change of ejector firing frequency for the pharmaceutical drug delivery device of FIG. 2.
FIG. 8 is a graphical representation of a change in fluid jet length versus a change of ejector firing frequency for the pharmaceutical drug delivery device of FIG. 2.
FIG. 9 is a plan view, not to scale, of a portion of an ejection head for the pharmaceutical drug delivery device of FIG. 2.
FIG. 10 is a graphical representation of a change in fluid plume height versus a change of time delay between fluid jet firing bursts for the pharmaceutical drug delivery device of FIG. 2.

### DETAILED DESCRIPTION

For the purposes of this disclosure, the following terms are defined:
a) plume means the randomly directed mist of fluid droplets with low inertia that are affected by air resistance and air currents and are likely to float throughout a nasal chamber for more even coverage;
b) plume angle is a measure of an angle of a cone-shaped volume of randomly directed mist of fluid droplets in the plume;
c) plume height is a measure of a total height of mist of fluid droplets in a plume measured from an outlet of a fluid ejection head to a total travel distance of the plume;
d) fluid jet length is a measure of a length of high inertia fluid droplets ejected from an outlet of an ejection head to the apex of the plume angle;
e) burst is defined as the number of times a fluid droplet is ejected from an individual nozzle. A burst of fluid occurs when a fluid ejector is fired by a series of voltage pulses of sufficient magnitude to eject fluid through an associated nozzle;
f) burst length is defined as the total number of times each of the fluid ejectors is fired per burst; and
g) burst delay is defined as amount of time between individual bursts.

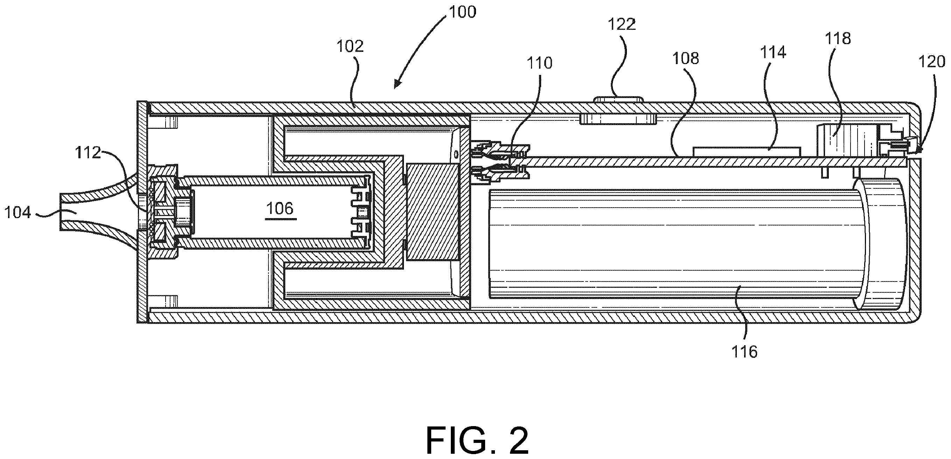

An illustration of a pharmaceutical drug delivery device 100 is illustrated in a cross-sectional view, not to scale, in FIG. 2. The device includes a a cartridge body 102, having a fluid outlet nozzle 104 attached to the cartridge body 102. A fluid jet ejection cartridge 106 is disposed in the cartrdige body 102. The fluid jet ejection cartridge 106 contains a liquid pharmaceutical drug to be administered by the device 100. A logic board 108 is disposed in cartridge body 102 and is electrically connected via a logic board connector 110 to an ejection head 112 on the fluid jet ejection cartridge 106. As described in more detail below, the ejection head 112 includes plurality of fluid ejection nozzles and associated fluid ejectors. A processor 114 is disposed on the logic board 108 or on the ejection head 112 for executing a control algorithm to control the ejection head 112 to modify plume characteristics of fluid ejected from the ejection head by controlling one or more operating parameters selected from (a) fluid jet firing frequency, (b) burst length, and (c) fluid jet firing burst delay. The cartridge body 102 may include a rechargeable battery 116 electrically connected to the logic board 108 for providing power to the ejection head 112. A power switch 118 is used to activate the device 100. A USB input 120 may also be included to reprogram the processor for use with different pharmaceutical fluids. An activation button 122 may be used to initiate delivery of the pharmaceutical drug on-demand by a user.

A wide variety of ejection heads 112 may be used with the device 100 described above. Accordingly, the ejection head 112 may be selected from a thermal jet ejection head, a bubble jet ejection head or a piezoelectric jet ejection head. Each of the foregoing ejection heads can produce a spray of fluid on demand and may be programmed to provide a variety of fluid plume characteristics as described below. By contrast, conventional spray pumps are mechanically fixed for a particular drug delivery application and generally cannot be modified to provide a variety of fluid plume characteristics.

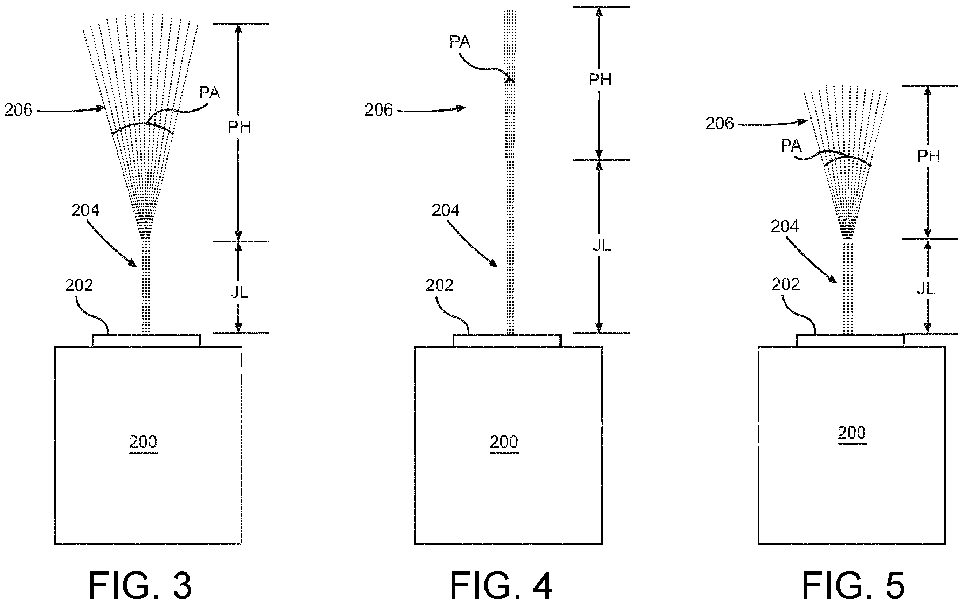

Unlike conventional inkjet ejection heads which are designed to eject fluid droplets in a straight line for 2 to 3 mm to reach a substrate such as paper, the device 100 described herein is designed to eject fluid droplets as a mist further into an air stream so that the droplets eventually land in the mucosa area of the nasal cavity. FIG. 3 is a schematic illustration of fluid jet ejection device 200 having an ejection head 202 for ejecting fluid therefrom to form a relatively high velocity fluid jet stream 204 and a low velocity plume 206 of fluid mist that floats on ambient air currents. The plume 206 characteristics, such as plume height PH and plume angle PA as well as the fluid jet stream length JL may be affected by how the ejection head 202 is operated. For example, a firing frequency of the ejection head can effect the plume height PH. A higher firing frequency results in a wider plume 206 and a shorer fluid jet stream length JL. The wider plume 206 may be the result of collisions between droplets as they are ejected from the ejection head 202. Another characteristics that effects the plume 206 is the entrainment of fluid droplets from the ejection head 202. High velocity fluid droplets tend to create an airflow perpendicular to the ejection head 202 which entrains subsequent droplets ejected from the ejection head 202 to draw the droplets further from the ejection head 202. Accordingly, air entrainment can affect both the fluid jet stream 204 and the plume 206.

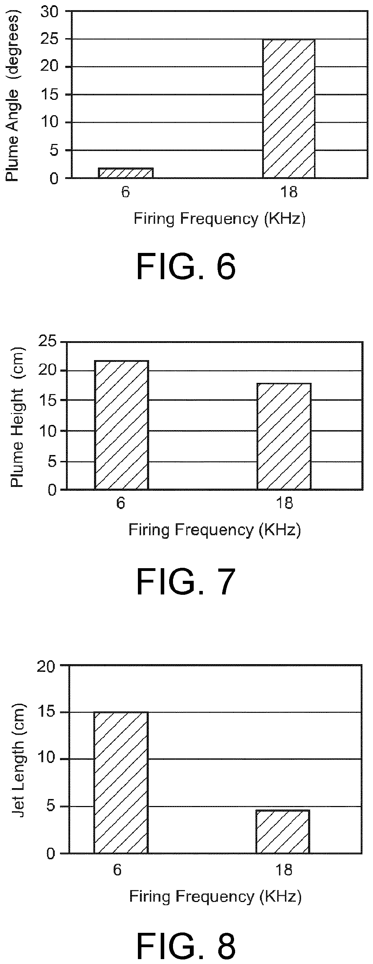

As shown in FIGs. 4-8, the firing frequency has an effect on the plume angle PA, plume height PH and fluid jet stream length JL for an ejection device 200 having an ejection head 202 with 96 fluid outlet nozzles. Each nozzle is sized to expell from about 2 to about 24 pL per droplet. In some embodiments, each nozzle is sized to expel about 4 pL per droplet. For example, in FIG. 4, the ejection device 200 was operated with a frequency of 6 KHz and resulted in a plume angle PA of about 5 degrees, a plume height PH of about 21.5 centimeters, and a fluid jet stream length JL of about 15 centimeters (FIGs. 6-8). In FIG. 5, the same ejection device 200 was operated with a frequency of 18 KHz and resulted in a plume angle PA of about 25 degrees, a plume height PH of about 18 cm, and a fluid jet stream length JL of about 4.5 cm (FIGs. 6-8). Accordingly, as demonstrated above, higher frequencies can be used to produce wider plumes 206 and shorter fluid jet streams 204 and lower frequencies can be used to produce narrower plumes 206 and longer fluid jet streams 204. A higher frequency may also be used to increase the dispense rate and reduce the time of delivery for a prescribed dose of a pharmaceutical drug.

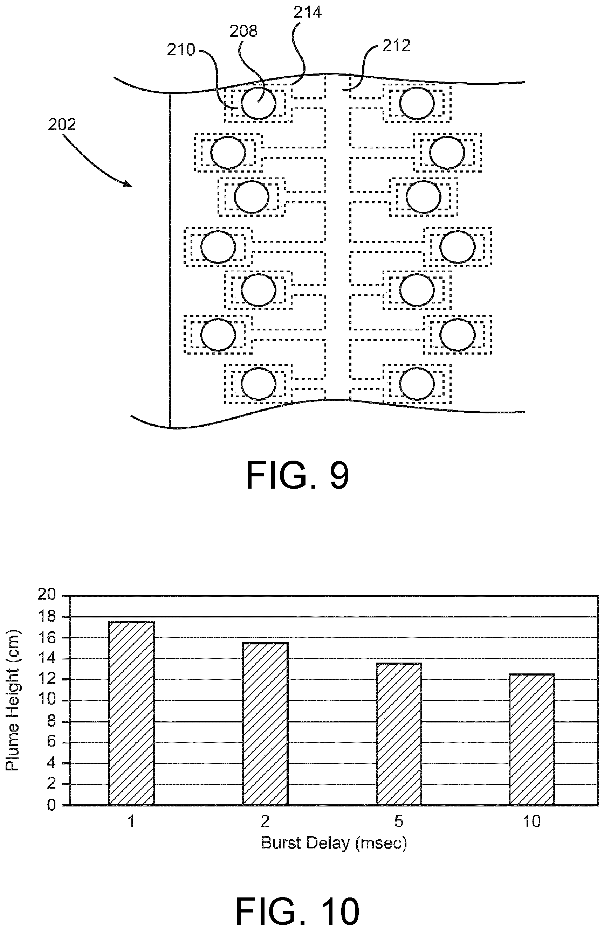

Another parameter that has an effect on the fluid jet stream and plume characteristics is the burst length. A portion of the ejection head 202 for ejecting 4 pL droplets per nozzle is illustrated in plan view in FIG. 9. The ejection head 202 contains a nozzle plate containing a plurality of fluid outlet nozzles 208 wherein each fluid outlet nozzle 208 is disposed above a fluid ejector 210. Fluid to be ejected through the fluid outlet nozzles 208 is supplied from a fluid cartridge through a fluid via 212 to an ejection chamber 214 containing the fluid ejector 210. It was demonstrated with the ejection device 200 containing an ejection head 202 with 96 fluid outlet nozzles 208 operated at 18 KHz that, as a total number of times each of the fluid ejectors 210 is activated per burst is increased, a distinct fluid jet stream 204 from the ejection head 202 can be produced. For example, activating each fluid ejector 25, 50, 75, 100 and 150 times per burst failed to produce a distinct fluid jet stream 204. However, activating each fluid ejector 200 times per burst provided a distinctly visible fluid jet stream. A distinct fluid jet stream 204 occurs when more fluid droplets are entrained in the fluid jet stream thereby increasing the dosage of fluid delivered in a plume originating from the jet stream.

Finally, it was demonstrated that the time delay between fluid jet firing bursts from 50 nozzles 208 of the ejection head 202 can be used to change the plume characteristics. As before, each nozzle was designed to eject 4 pL droplets of fluid per burst. As shown in FIG. 10, as the burst delay was increased from 1 to 10 milliseconds, the plume height PH decreased from about 17 cm to about 12 cm. Likewise, the plume angle PA decreased from about 27 degrees to about 18 degrees. It is believed that with a burst delay of 10 milliseconds, there is still droplet entrainment. However the entrainment decreases as the burst delay increases thereby reducing the plume height PH and plume angle PA. Typical burst delays may range from 0 to 15 milliseconds. With zero burst delay, there is one continuous burst to eject fluid from the ejection head. It will be appreciated that with longer firing burst delays, the time increases for delivering a predetermine dose of pharmaceutical drug to a patient. Accordingly, an advantage of the disclosed embodiments is a fluid jet ejection head can be tuned to administer a variety of drugs from a pharmaceutical drug delivery device to achieve multiple plume characteristics for broader applications for nasal delivery of drugs. Plume control can be achieved by changing one or more operating parameters selected from of (a) fluid jet firing frequency, (b) burst length, and (c) fluid jet firing burst delay. Accordingly, combinations of operating parameters such as (a) fluid jet firing frequency and (b) bursth length; (a) fluid jet firing frequency and (c) fluid jet burst delays; (b) burst length and (c) fluid jet firing burst delays, or a combination of all three operating parameters (a), (b) and (c) may be used to change the plume and jet characteristics of the fluid droplets expelled from the drug delivery device. Furthermore, sequencing the operating parameters (a), (b) and (c) in any order may be used to deposit fluid in different parts of the nasal cavity. Such changes can be used to tune the device without the need for external mechanisms to control plume characteristics in order to deposit predetermined amounts of fluid in different parts of the nasal cavity.

It is noted that, as used in this specification and the appended claims, the singular forms "a," "an," and "the," include plural referents unless expressly and unequivocally limited to one referent. As used herein, the term "include" and its grammatical variants are intended to be nonlimiting, such that recitation of items in a list is not to the exclusion of other like items that can be substituted or added to the listed items

For the purposes of this specification and appended claims, unless otherwise indicated, all numbers expressing quantities, percentages or proportions, and other numerical values used in the specification and claims, are to be understood as being modified in all instances by the term "about." Accordingly, unless indicated to the contrary, the numerical parameters set forth in the following specification and attached claims are approximations that can vary depending upon the desired properties sought to be obtained by the present disclosure. At the very least, and not as an attempt to limit the application of the doctrine of equivalents to the scope of the claims, each numerical parameter should at least be construed in light of the number of reported significant digits and by applying ordinary rounding techniques.

While particular embodiments have been described, alternatives, modifications, variations, improvements, and substantial equivalents that are or can be presently unforeseen can arise to applicants or others skilled in the art. Accordingly, the appended claims as filed and as they can be amended are intended to embrace all such alternatives, modifications variations, improvements, and substantial equivalents.

## Claims

1. A pharmaceutical drug delivery device (100, 200) comprising:
a cartridge body (102);
a fluid outlet nozzle (104, 208) attached to the cartridge body (102);
a fluid jet ejection cartridge (106) disposed in the cartridge body (102), the fluid jet ejection cartridge (106) containing a liquid pharmaceutical drug and an ejection head (112, 202) containing a plurality of fluid ejection nozzles and associated fluid ejectors (210) is attached to the fluid jet ejection cartridge (106); and
a processor (114) disposed on a logic board (108) or the ejection head (112, 202) for executing a control algorithm to control the ejection head (112, 202) to modify plume characteristics of fluid ejected from the ejection head (112, 202) by controlling one or more operating parameters selected from the group consisting of (a) fluid jet firing frequency, (b) burst length, and (c) fluid jet firing burst delay.

2. The pharmaceutical drug delivery device (100, 200) of claim 1, wherein each fluid droplet ejected from the pharmaceutical drug delivery device (100, 200) has volume ranging from about 2 to about 24 pL.

3. The pharmaceutical drug delivery device (100, 200) of claim 1, wherein the ejection head (112, 202) has a firing frequency ranging from about 2 to about 20 KHz.

4. The pharmaceutical drug delivery device (100, 200) of claim 1, wherein the burst length ranges from about 20 to about 250 times per burst.

5. The pharmaceutical drug delivery device (100, 200) of claim 1, wherein the fluid jet firing burst delay ranges from about 0 milliseconds to about 15 milliseconds.

6. A method of controlling a fluid plume from a fluid ejection device (100, 200) for delivery of pharmaceutical drugs, the method comprising:
providing the fluid ejection device (100, 200) comprising a cartridge body (102), a fluid outlet nozzle (104, 208) attached to the cartridge body (102) and a fluid jet ejection cartridge (106) disposed in the cartridge body (102), the fluid jet ejection cartridge (106) containing a liquid pharmaceutical drug, wherein a fluid ejection head (112, 202) containing a plurality of fluid ejection nozzles and associated fluid ejectors (210) is attached to the fluid jet ejection cartridge (106) and the fluid ejection head (112, 202) is in fluid flow communication with the fluid outlet nozzle (104, 208),
providing a processor (114) in electrical communication with the fluid ejection head (112, 202),
configuring the processor (114) to execute a control algorithm to select one or more operating parameters selected from the group consisting of (a) fluid jet firing frequency, (b) burst length, and (c) fluid jet firing burst delay in order to modify fluid plume characteristics of fluid ejected from the ejection head (112, 202) through the fluid outlet nozzle (104, 208), and
activating the fluid ejection device (100, 200) to deliver the pharmaceutical drug to a patient.

7. The method of claim 6, wherein the pharmaceutical drug is ejected from the fluid ejection device (100, 200) with a fluid plume angle ranging from about 25 to about 60 degrees.

8. The method of claim 6, wherein the pharmaceutical drug is ejected from the fluid ejection device (100, 200) with a fluid plume height ranging from about 10 to about 25 centimeters.

9. The method of claim 6, wherein the pharmaceutical drug is ejected from the fluid ejection device (100, 200) with a fluid jet length ranging from about 1 to about 25 centimeters from the fluid ejection head (112, 202).

10. The method of claim 6, wherein the pharmaceutical drug is ejected with a plume characteristic that delivers the pharmaceutical drug to turbinate areas of a nasal cavity (10) of the patient.

11. The method of claim 6, wherein the pharmaceutical drug is ejected with a plume characteristic that evenly distributes the pharmaceutical drug throughout a nasal cavity (10) of the patient.

12. The method of claim 6, wherein the pharmaceutical drug is ejected with a plume characteristic that increases a drug dose delivery rate to the patient.

13. A method for nasal cavity injection of pharmaceutical drugs, comprising:
providing a fluid ejection device (100, 200) comprising a cartridge body (102), a fluid outlet nozzle (104, 208) attached to the cartridge body (102) and a fluid jet ejection cartridge (106) disposed in the cartridge body (102), the fluid jet ejection cartridge (106) containing a pharmaceutical drug, wherein a fluid ejection head (112, 202) containing a plurality of fluid ejection nozzles and associated fluid ejectors (210) is attached to the fluid jet ejection cartridge (106) and the fluid ejection head (112, 202) is in fluid flow communication with the fluid outlet nozzle (104, 208),
providing a processor (114) in electrical communication with the fluid ejection head (112, 202),
configuring the processor (114) to execute a control algorithm to select one or more operating selected from the group consisting of (a) fluid jet firing frequency, (b) burst length, and (c) fluid jet firing burst delay in order to modify fluid plume characteristics of fluid ejected from the ejection head (112, 202) through the fluid outlet nozzle (104, 208), and
activating the fluid ejection device (100, 200) to deliver the pharmaceutical drug in the nasal cavity (10) of a person.

14. The method of claim 13, wherein the pharmaceutical drug is ejected with a plume characteristic that delivers the pharmaceutical drug to turbinate areaa of the nasal cavity (10).

15. The method of claim 13, wherein the pharmaceutical drug is ejected with a plume characteristic that evenly distributes the pharmaceutical drug throughout the nasal cavity (10).

16. The method of claim 13, wherein the pharmaceutical drug is ejected with a plume characteristic that increases a drug dose delivery rate to the nasal cavity (10).

17. The method of claim 13, wherein the pharmaceutical drug is ejected from the fluid ejection device (100, 200) with a fluid plume angle ranging from about 25 to about 60 degrees.

18. The method of claim 13, wherein the pharmaceutical drug is ejected from the fluid ejection device (100, 200) with a fluid plume height ranging from about 10 to about 25 centimeters.

19. The method of claim 13, wherein the pharmaceutical drug is ejected from the fluid ejection device (100, 200) with a fluid jet length ranging from about 1 to about 25 centimeters from the fluid ejection head (112, 202).
